# EUROPÄISCHE PATENTSCHRIFT

(11) **EP 0 960 945 B1**
(45) Veröffentlichungstag und Bekanntmachung des Hinweises auf die Patenterteilung: **04.09.2002**
(21) Anmeldenummer: 99109414.5
(22) Anmeldetag: 11.05.1999
(51) Int. Cl.: C12P 35/04, C12P 37/04

(54) **Verfahren zur enzymatischen Herstellung von Betalaktamantibiotika mit Produktrückgewinnung bei pH 1 in Gegenwart von immobilisiertes Penicillinamidase**
Enzymatic process for producing beta-lactam antibiotics involving product recovery at pH 1 in the presence of immobilized penicillin amidase
Procédé enzymatique pour la production d'antibiotiques bêta-lactame avec récupération de produit à pH 1 en présence de pénicilline amidase immobilisée

(30) Priorität: 26.05.1998 DE 19823332
(43) Veröffentlichungstag der Anmeldung: 01.12.1999
(73) Patentinhaber: Unifar Kimya Sanayii ve Ticaret A.S, 80290 Gayrettepe, Istanbul (TR)
(72) Erfinder: Ilhan, Ferhat, 81120 Atasehir, Istanbul (TR); Kraemer, Dieter, 55116 Mainz (DE)
(74) Vertreter: Holtfoth, Hans-Jürgen

(56) Entgegenhaltungen:
- WO-A-93/12250
- HAUFLER ET AL.: "Structure, pH-stability and renaturation of free and immobilized E. coli penicillin amidase" DECHEMA BIOTECHNOLOGY CONFERENCES, Bd. 1, 1988, Seiten 345-350, XP002120269
- TISCHER W ET AL: "Immobilized enzymes: crystals or carriers?" TRENDS IN BIOTECHNOLOGY, Bd. 17, Nr. 8, August 1999 (1999-08), Seite 326-335 XP004172537 ISSN: 0167-7799

## Beschreibung

Es ist seit längerem bekannt, daß sich Betalactam-Antibiotika aus ihrer jeweiligen Kern- und Seitenkettenkomponente auf enzymatischem Wege bilden können (C. A. Claridge & al., Nature, Vol.187; 237, **1960**).
Aus neueren Untersuchungen ist bekannt, daß ein auf enzymatischem Wege hergestelltes Betalactam-Antibiotikum, z.B. Amoxicillin, eine höhere Reinheit und dementsprechend eine geringere Toxizität aufweist als auf chemischem Wege hergestelltes Amoxicillin (PCT WO 94/17800).
Entsprechend groß ist das allgemeine Interesse an industriell sinnvollen Verfahren zur enzymatischen Herstellung von Betalactam-Antibiotika.
Hierzu müssen eine Reihe technischer Probleme gelöst sein, vor allem:
a) das Problem, die von dem Enzym katalysierte erwünschte Synthesereaktion so hoch wie möglich, und die von demselben Enzym katalysierten unerwünschten Nebenreaktionen, nämlich die Hydrolyse der aktivierten Seitenkettenkomponente sowie des Betalactam-Antibiotikums, so gering wie möglich zu halten (siehe Formelschema). 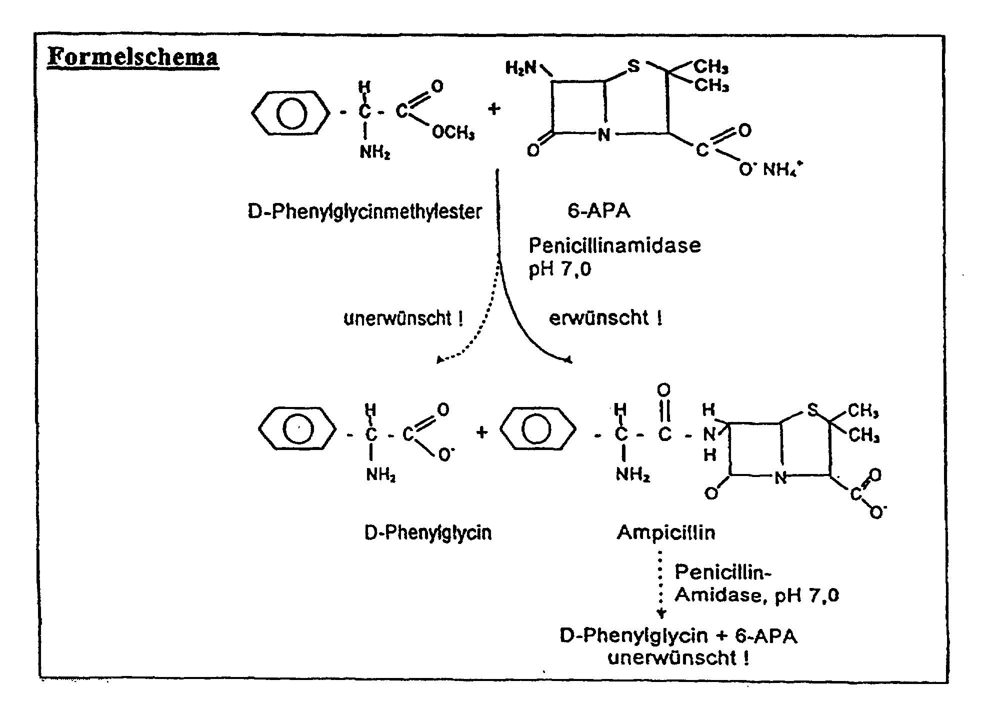
b) das Problem, das Produkt zu isolieren in einer Weise, die die Wiederverwendbarkeit des Biokatalysators, d.h. des Enzyms, erlaubt.

Nun gibt es bereits eine Reihe von Patentanmeldungen und bereits erteilten Patenten, die entscheidende Fortschritte bei der Lösung dieser Probleme beanspruchen, auf der Basis experimenteller Befunde, die mit an unlösliche Trägerpartikel meist kovalent immobilisierten Enzymen gewonnen wurden; also mit Systemen, die zur Wiederverwendung von Enzymen in den letzten Jahrzehnten entwickelt worden sind.
Es sollte daher selbstverständlich gewesen sein, die operationale Stabilität (O-ST) dieser immobilisierten Enzyme unter Anwendungsbedingungen untersucht zu haben, um eine Vorstellung von der industriellen Produktivität eines solchen Biokatalysators zu gewinnen.
In den meisten Fällen begnügte man sich aber mit einem Minimum an Untersuchungen, nämlich der Untersuchung einer einzigen Umsetzung.
Das hierbei erzielte Resultat wurde dann oft in einen globalen Anspruch generalisiert, ohne Rücksicht auf einen dem Experten wohlbekannten Fakt, daß zur Charakterisierung eines immobilisierten Enzyms und einer von ihm katalysierten Reaktion ein nur aus einer einzigen Umsetzung gewonnenes Ergebnis meist irreführend ist, und zwar aus den folgenden Gründen:
1. Die Aktivität, die man für die erste Verwendung eines kovalent immobilisierten Enzyms mißt, ist meist viel zu hoch, weil oft noch freies Enzym vorhanden ist. Dieses freie Enzym ist nicht-kovalent an das Trägermaterial adsorbiert und täuscht so eine höhere Aktivität des "immobilisierten" Enzyms vor, und zwar so lange, bis es durch Wechselwirkung mit dem Substrat und durch Waschprozesse beseitigt ist, was mindestens einen Verwendungszyklus, meist aber deren mehrerer bedarf.
   In diesem Zusammenhang sei nochmals daran erinnert, daß immobilisierte Enzyme hauptsächlich aus Gründen ihrer Wiederverwendbarkeit, d.h. ihrer Verwendbarkeit über viele Umsetzungszyklen, sowie wegen der Reinheit der mit ihrer Hilfe hergestellten Produkte eingesetzt werden.
   Das Reinheitsgebot schließt Verunreinigungen durch freies Enzym selbstverständlich aus; ergo sollte das Produkt aus der ersten Umsetzung zur Herstellung von Arzneimitteln ohnehin nicht verwendet werden.
2. Substrat- und Produktmoleküle können an das Trägermaterial, an welchem das Enzym kovalent immobilisiert ist, adsorbieren und so der analytischen Bestimmung entgehen, so daß eine falsche Bilanz bezüglich der Synthese- und Hydrolysereaktion resultiert.
   Dieses Phänomen ist vor allem für die erste Umsetzung relevant. Danach kommt es über Absättigung der adsorbierenden Trägeroberfläche zu einer Art stabilem Zustand. In diesem Kontext sind analytische Methoden, die nur den gelösten Anteil berücksichtigen, irreführend.
   Für eine korrekte Bilanzierung sollten nur Methoden eingesetzt werden, welche die Solubilisierung aller vorhandenen Moleküle ermöglichen.
3. Das immobilisierte Enzym verliert fast immer im Laufe seiner Verwendung an Aktivität, besonders aber während der ersten drei Verwendungszyklen.

Aus allen diesen Gründen ist es geboten, mindestens vier aufeinanderfolgende Umsetzungszyklen (Batch-Zyklen) zu fahren, um eine einigermaßen korrekte Abschätzung von Ausbeute an Produkt, Konversionsrate der Reaktion und O-ST des Biokatalysators zu erhalten.
Die Untersuchungsmethoden müssen selbstverständlich signifikant sein, d.h. das Produkt muß, wenn möglich, isoliert und auf seine Zusammensetzung z.B. mit HPLC oder NMR untersucht werden. Proben, die aus der heterogenen Produktoder Reaktionsmischung gezogen werden, müssen vor ihrer Untersuchung solubilisiert werden.

Diese Regeln wurden jedoch in den meisten der Patentanmeldungen bzw. bereits erteilten Patenten nicht beachtet.
Nach unserem besten Wissen existiert gegenwärtig nur eine einzige Anmeldung (PCT WO 96 23897, Chemferm, Boesten & al.), welche Daten zur O-ST des Biokatalysators durch Untersuchung von fünf aufeinanderfolgenden Batch-Zyklen gibt, bei einer Konversionsrate von ca. 40 % der Kernkomponente.

Ein anderer kritischer Punkt auf dem Gebiet der enzymatischen Synthese von Betalactam-Antibiotika betrifft die Isolierung des Produktes.
Sie ist aus folgenden Gründen problematisch:

Das so hergestellte Antibiotikum fällt gewöhnlich als heterogenes Gemisch von festem und gelöstem Produkt an, welches von dem ebenfalls festen Biokatalysator, dem immobilisierten Enzym, abgetrennt werden muß. Hierzu veröffentlichten Clausen & al (PCT WO 960 2663 A1) einen Prozeß zur Herstellung von Betalactam-Antibiotika bei konstant hoher Konzentration der Reaktionsteilnehmer, wobei das gewünschte Produkt hauptsächlich als Festprodukt in Form von kleinen Kristallen anfällt, welche kontinuierlich aus dem Reaktor abgetrennt werden, indem man sie durch ein Sieb, welches den Biokatalysator zurückhält, in eine Zentrifuge leitet, wo diese Kristalle isoliert werden.
Der Zentrifugier-Überstand wird über einen Tank, in dem weiteres Substrat zugegeben wird, wieder in den Reaktor gepumpt. Dieser Prozeß bietet einen bemerkenswerten Fortschritt, nämlich das Produkt wird via Kristallisation und Zentrifugation rasch aus dem Reaktionsmedium entfernt und kann folglich nicht zu dem unerwünschten Nebenprodukt hydrolysieren.
Trotz dieses Vorteils erlaubt das Verfahren einen problemlosen Dauerbetrieb im Rührreaktor nicht.
Es besteht das Risiko, daß bei diesem Prozeß, der im Grunde genommen ein Naßsiebprozeß ist, wegen der Uneinheitlichkeit der Korngrößen das Bodensieb des Reaktors allmählich verstopft wird, und daß sich im Reaktor selbst durch Ansammlung fester Produkte zunächst eine pastöse Masse und schließlich ein festes Konglomerat von Produkt- und Katalysatorteilchen bildet, welches den Weiterbetrieb des Reaktors unmöglich macht.
Zusammenfassend läßt sich sagen, daß der vor dieser Erfindung bekannte Stand der Technik das eigentliche Problem, nämlich eine saubere Abtrennung des Produktes bei Wiederverwendbarkeit, d.h. Weiterverwendbarkeit des Biokatalysators ungelöst ließ.
Es besteht ein Bedarf nach einem solchen Verfahren, welches sich problemlos im Rühr-Reaktor oder im Festbett-Reaktor durchführen läßt.

Ziel dieser Erfindung ist die Realisierung eines solchen Verfahrens.

Gelöst wird die Aufgabe nach der Erfindung durch ein Verfahren zur Herstellung von aminogruppenhaltigen Betalactam-Antibiotika aus ihren Aminobetalactam-Komponenten und den entsprechenden aminogruppenhaltigen acylierenden Seitenkettenkomponenten über die biokatalytische Wirkung eines an Trägerpartikel kovalent immobilisierten Enzyms, wobei die Reaktionsprodukte bei pH 1,0 und in einem Temperaturbereich von 0°C bis +5°C in Gegenwart des immobilisierten Enzyms Penicillinamidase aus E. coli solubilisiert werden und dann von dem immobilisierten Enzym abgetrennt werden, welches anschließend durch Behandlung mit einem Puffer neutralen pH-Bereichs bei einer Temperatur von ca. +5°C in Bezug auf seine Aktivität regeneriert wird.

Besonders vorteilhaft ist es dabei, wenn das herzustellende Antibiotikum Ampicillin, die Aminobetalactam-Komponente 6-Aminopenicillansäure und die acylierende Seitenkettenkomponente D-Phenylglycin-Methylester oder D-Phenylglycinamid ist.

Bei einer anderen Ausführung des Verfahrens ist das herzustellende Betälactam-Antibiotikum Cephalexin, die Aminobetalactam-Komponente 7-Amino-Desacetoxycephalosporansäure (7-ADCA) und die acylierende Seitenkettenkomponente D-Phenylglycin-Methylester oder D-Phenylglycinamid.

Bei einer weiteren Ausführung des Verfahrens ist das herzustellende Betalactam-Antibiotikum Cefaclor, die Aminobetalactam-Komponente 3-Chloro-7-Amino-Desacetoxycephalosporansäure und die acylierende Seitenkettenkomponente D-Phenylglycin-Methylester oder D-Phenylglycinamid.

Bei einer anderen Ausführung des Verfahrens ist das herzustellende Betalactam-Antibiotikum Amoxicillin, die Aminobetalactam-Komponente 6-Aminopenicillansäure und die acylierende Seitenkettenkomponente D-p-Hydroxyphenylglycin-Methylester oder D-p-Hydroxyphenylglycinamid.

Bei einer weiteren Ausführung des Verfahrens ist das herzustellende Betalactam-Antibiotikum Cefadroxil, die Aminobetalactam-Komponente 7-Amino-Desacetoxycephalosporansäure (7-ADCA) und die acylierende Seitenkettenkomponente D-p-Hydroxyphenylglycin-Methylester oder D-p-Hydroxyphenylglycinamid.

Weiterhin ist für das Verfahren von Bedeutung, daß die Trägerpartikel eine makroporöse Struktur bei einem Teilchendurchmesser von 10-1000 Micrometer haben.

Vorteilhafterweise haben die Trägerpartikel Oxirangruppen zur kovalenten Immobilisierung des Enzyms.

Besonders vorteilhaft ist es, wenn die oxirangruppenhaltigen makroporösen Trägerpartikel aus synthetischen Polymeren bestehen.

Es ist aber auch möglich, daß die oxirangruppenhaltigen makroporösen Trägerpartikel aus anorganischem Material bestehen.

Ebenfalls möglich ist, daß die anorganischen Trägerpartikel aus porösem Glas bestehen.

Verfahrenswesentlich ist, daß alle verfahrensrelevanten Schritte in einem Rührreaktor ausgeführt werden, der mit einem Bodensieb versehen ist.

Es ist aber auch möglich, daß alle verfahrensrelevanten Schritte in einem Festbettreaktor durchgeführt werden.

Das Verfahren ist weiterhin dadurch gekennzeichnet, daß zur Solubilisierung der Reaktionsprodukte fünfprozentige bis fünfzigprozentige Schwefelsäure in einer Menge zu dem gekühlten Reaktionsgemisch zugegeben wird, bis in diesem ein pH-Wert von 1,0 erreicht ist.

Das Verfahren ist weiterhin dadurch gekennzeichnet, daß der zur Regenerierung des immobilisierten Enzyms verwendete Puffer Phosphatpuffer von einer Molarität von 0,1 bis 1,0 und einem pH-Wert von 6,5 bis 8,0 ist.

Am Beispiel des Betalactam-Antibiotikums Ampicillin wurde gefunden, daß das Problem einer sauberen Trennung von enzymatisch hergestelltem Ampicillin und dem hierzu verwendeten immobilisierten Enzym gelöst werden kann, indem man eine starke Säure zu der Suspension von Produkt und immobilisiertem Enzym bei einer Temperatur von 0°C bis +2°C zugibt, bis ein pH-Wert von 1,0 erreicht ist. Bei diesem pH kommt es zu einer raschen Auflösung aller Reaktionsprodukte. Die so solubilisierten Produkte können leicht abgetrennt werden; im Falle eines Rührreaktors durch das Bodensieb des Reaktors, im Falle eines Festbettreaktors durch Elution, wobei das immobilisierte Enzym im jeweiligen Reaktor verbleibt. Es wird durch die Säurebehandlung fast vollständig, das heißt zu mehr als 90%, inaktiviert.
Nun wurde desweiteren gefunden, daß das so inaktivierte Enzym fast vollständig, d.h. zu mehr als 90 %, reaktiviert werden kann, indem man einen Puffer neutralen pH-Wertes zu dem inaktivierten immobilisierten Enzym (I-E) gibt und das in diesem Puffer suspendierte I-E mehrere Stunden bei einer Temperatur von ca. + 5°C suspendiert.

Diese Regenerierbarkeit des I-E nach Totalverlust seiner Aktivität ist das vom Fachmann unerwartete Ergebnis dieser Erfindung. Die erfindungsgemäße Regenerierbarkeit des I-E bleibt auch nach mehreren Umsetzungen und den damit verbundenen mehreren Säurebehandlungen voll erhalten.

Diese Methode funktioniert mit dem Enzym Penicillinamidase aus E. coli (E.C. 3.5.1.11.) welches an makroporöse Perlen aus Polymethacrylat-Komponenten kovalent immobilisiert ist. Es ist wahrscheinlich, daß diese Methode auch mit Penicillinamidasen aus anderen Microorganismen (z. B. aus Bazillus Megatherium oder aus Xanthomonas Citrii) funktioniert.

Die Methode sollte mit jedem Betalactam-Antibiotikum (BLA) funktionieren, welches eine freie Aminogruppe in der Seitenkette besitzt, z. B. mit Amoxicillin, Cephalexin, Cephadroxil, Cefaclor oder Cefroxadin.

Im folgenden werden weitere Erläuterungen zu folgenden Punkten gegeben:
Immobilisiertes Enzym (I-E)
Trägermaterial
Aktivitätsverlust von I-E im sauren pH-Bereich
Regenerierung der Aktivität von I-E
Regenerierbarkeit: ein Novum gegen den bisherigen Stand der Technik
Reaktoren zur Durchführung des erfindungsgemäßen Verfahrens
Fazit

### Das immobilisierte Enzym (I-E)

Für die enzymatische Synthese des Betalactam-Antibiotikums Ampicillin wurde von uns das Enzym Penicillinamidase aus E. coli (E. C. 3.5.1.11.) kovalent gebunden an Eupergit C® eingesetzt. Ein solcher Biokatalysator wird zum Beispiel von der Firma FZB (Berlin) hergestellt und unter dem Namen PharmaKAT-PcA zum Verkauf angeboten. Bei unseren Versuchen haben wir hauptsächlich dieses Produkt verwendet.

### Das Trägermaterial

Eupergit C® ist ein perlförmiges Trägermaterial zur kovalenten Immobilisierung von Enzymen. Es wird von der Firma Röhm GmbH (D-64293 Darmstadt) hergestellt und in den Handel gebracht. Der Teilchendurchmesser von Eupergit C® beträgt 50 - 250 micron. Es ist ein Copolymeres aus Methacrylamid, N.N'-Methylen-Bis-Methacrylamid, Glycidylmethacrylat und Allylglycidyläther. Enzyme können kovalent an Eupergit C® gebunden werden, wobei es unter anderem zwischen den Aminogruppen des Enzyms und den Oxiran-Gruppen der Glycidylkomponenten des Eupergit C® zu kovalenten Bindungen kommt. Diese Bindung ist stabil gegen Säuren, so daß es bei der erfindungsgemäßen Säurebehandlung nicht zu einer Ablösung des Enzyms aus dem Immobilisat kommt. Eupergit C® selbst zeigt eine ausreichende Säurestabilität, d. h., es ist auch bei längerer Einwirkung von Säure formstabil und behält sein Bettvolumen bei, d. h., man beobachtet weder Quellung noch Schrumpfung des Immobilisates bei pH-Änderungen von pH 7,5 auf pH 1,0 und vice versa. Dies ist vor allem für die erfindungsgemäße Anwendung im Festbettreaktor sehr wichtig. Anstelle von Eupergit C® wäre jedes andere Trägermaterial, welches die obengenannten Kriterien erfüllt, ebenfalls für das erfindungsgemäße Verfahren geeignet, beispielsweise Partikel aus porösem Glas, falls entsprechend mit Gruppen derivatisiert, welche säurestabil sind und Ausbildung von säurestabilen kovalenten Bindungen zum Enzymmolekül ermöglichen.

### Aktivitätsverlust von I-E im sauren pH-Bereich

An Eupergit C® kovalent gebundene Penicillinamidase war vor Jahren von der heute nicht mehr existenten Firma Röhm Pharma hergestellt und unter dem Handelsnamen Eupergit-PcA vertrieben worden. In einem anwendungstechnischen Merkblatt bezüglich der Verwendung dieses Biokatalysators zur Herstellung von 6-Aminopenicillansäure wurde auf den Aktivitätsverlust des immobilisierten Enzyms im sauren pH-Bereich hingewiesen und Maßnahmen zur Minimierung dieses Risikos empfohlen.

Erwartungsgemäß kam es zu einem fast vollständigen Aktivitätsverlust (> 95%), wenn wir kalte 12,5 - 25%ige Schwefelsäure zu einer gekühlten, wässrigen Suspension von PharmaKAT-PcA und dem Produktgemisch der enzymatischen Ampicillinsynthese zugaben, um das ungelöste Ampicillin bei pH 1,0 in Lösung zu bringen, um es von den Biokatalysator-Teilchen abfiltrieren zu können. Diese Art der Solubilisierung und Abtrennung des Ampicillins aus dem Produktgemisch erwies sich als eine effiziente und leicht durchzuführende Methode, jedoch - wie erwartet - zum Preis des nahezu vollständigen Verlustes der Aktivität des Biokatalysators.

Die Solubilisierung von aminogruppenhaltigen Betalactam-Antibiotika aus ihrem Produktgemisch durch Ansäuern nach Abtrennung vom Biokatalysator (d.h. in dessen Abwesenheit!) war bekannt gewesen (US 54 70 717A).

### Regenerierung der Aktivität des durch Säure inaktivierten Enzyms

Zu unserer Überraschung konnte der solcherart inaktiv gewordene Biokatalysator fast vollständig regeneriert werden, d. h. seine ursprüngliche enzymatische Aktivität zurückgewinnen, wenn er in 0,1 molarem Kalium-Phosphatpuffer bei pH 7,5 für einen Zeitraum von 8 - 30 Stunden bei ca. +5 °Celsius inkubiert wurde.

Wir führten diese Prozedur stets mit derselben immobilisierten Enzymprobe viermal hintereinander, d. h. über vier konsekutive Synthesezyklen, ohne nennenswerten Verlust an Enzymaktivität durch, wobei unter Enzymaktivität die Menge an synthetisiertem Produkt pro Synthesezeit und eingesetzter Enzymmenge zu verstehen ist. Hierbei wurde jedesmal nach dem Syntheseschritt die Produktsuspension mit Schwefelsäure bei 0 °C auf pH 1,0 gebracht; danach die Produktlösung abfiltriert; das auf dem Filter im Reaktor verbleibende I-E zuerst mit etwas Wasser gewaschen und dann zu diesem I-E Puffer zugegeben und das Gemisch 16 Stunden lang bei pH 7,5 und ca. +5 °C inkubiert. Weitere experimentelle Details und Ergebnisse sind in den Beispielen gegeben.

In diesen Beispielen wird die Anwendbarkeit des erfindungsgemäßen Verfahrens auf die enzymatische BLA-Synthese im Rührreaktor und im Festbett-Reaktor demonstriert. Aus den Ergebnissen kann eine operationale Stabilität (O-ST) des I-E bei erfindungsgemäßer Durchführung von mehr als vier Synthesezyklen gefolgert werden.

### Regenerierbarkeit von I-E nach Inaktivierung durch Säure: ein NOVUM gegen den bisherigen Stand der Technik

Die erfindungsgemäße Regenerierbarkeit von I-E nach dessen fast vollständiger Inaktivierung durch Säure war aus dem bisherigen Stand der Technik nicht schlußzufolgern gewesen. Es war bekannt gewesen, daß das freie Enzym nach Inaktivierung bei pH 2,0 durch Inkubation in neutralem Puffer zu etwa 40% seiner ursprünglichen Aktivität regeneriert werden konnte, während die Reaktivierbarkeit des an Eupergit C® immobilisierten Enzyms demgegenüber "vermindert" war (U. Haufler, Dissertation, Seite 103, Universität Bremen, 1987 sowie Haufler et al., DECHEMA Biotechnol. Conf. (1988), Volume Date 1987,1., 345-350). Aber eine solche Verminderung der Regenerierbarkeit konnte in unseren Untersuchungen nicht bestätigt werden. Im Gegenteil, selbst nach vier konsekutiven Inaktivierungen bei einem Säurestress, d. h. bei einer Hydroniumionen-Konzentration, die zehnmal höher war als bei den Versuchen von U. Haufler und dazu noch viermal wiederholt wurde, konnte das so behandelte I-E zu fast 100% (> 95%) seiner ursprünglichen Aktivität und O-ST in Bezug auf enzymatische Ampicillinsynthese reaktiviert werden.

### Reaktoren zur Durchführung des erfindungsgemäßen Verfahrens

Wie bereits erwähnt, kann das erfindungsgemäße Verfahren in jedem Reaktortyp durchgeführt werden, der sich für den Einsatz von immobilisierten Enzymen zu biokatalytischen Zwecken eignet.

Bei Verwendung eines Rührkessel-Reaktors muß dieser mit einem Bodensieb oder mit Filterkerzen ausgerüstet sein, um den Biokatalysator, d. h. das I-E, im Reaktor zurückzuhalten, und zwar bei allen Einzelschritten der Durchführung, d. h. im einzelnen: während der enzymatischen Synthese sowie während der Solubilisierung des Produktes durch Säurezugabe, Abtrennung des Produktes durch Filtration, Regenerierung des immobilisierten Enzyms durch Inkubation in Puffer sowie Wiederbeschickung des Reaktors mit Substratlösung für den nächstfolgenden Zyklus.

Alle diese Schritte lassen sich bequem in demselben Reaktor, sozusagen als Eintopf-Verfahren, durchführen.

Als Festbett-Reaktoren (FBR) eignen sich Säulen für die Präparative Chromatographie (im Produktionsmaßstab), wie sie von einer Reihe von Firmen (z. B. der Firma Merck/Darmstadt) angeboten werden, sofern diese aus säurestabilen Materialien gefertigt sind und über säurestabile Zufuhr- und Abfuhrschläuche verfügen, deren innerer Durchmesser groß genug ist, um relativ hohe Flußraten zu ermöglichen. Außerdem müssen die Säulen mit einem Temperiermantel versehen sein, durch den man Kühlflüssigkeit leiten kann. Außerdem sind die Standardsysteme mit Vorrichtungen zu ergänzen, die es erlauben, Säure oder Puffer durch die Säule zu rezirkulieren und durch zusätzliche externe Kühlsysteme effizient zu kühlen. Desweiteren benötigt man für den Betrieb und die Kontrolle des FBR geeignete Pumpen sowie Meßinstrumente für Druck, Temperatur und Flußrate. Systeme, die eine automatische Steuerung ermöglichen, sind wünschenswert.

Ein FBR wird, wie folgt, betrieben:

Die Substratlösung wird kontinuierlich bei konstanter Flußrate durch die FBR-Säule (in der sich I-E befindet) gepumpt, und das dort gebildete Produkt wird am Ausgang der Säule in einem Tank zur weiteren Aufreinigung gesammelt. Sobald die Flußrate abnimmt bzw. der Druck in der Säule ansteigt - ein Hinweis, daß die Säule allmählich durch Produktkristalle verstopft -, wird die Substratzufuhr unterbrochen und die Zufuhr von Säure gestartet, die durch Durchleiten durch ein Kühlbad auf 0° C gebracht wurde. Dieser Säurestrom wird kontinuierlich durch die Säule, die über ihren Temperiermantel ebenfalls gekühlt wird, rezirkuliert, bis sich alles Festprodukt in der Säure aufgelöst hat, was man z. B. daran erkennt, daß man dieselbe Flußrate wie zu Anfang der Substratzugabe erreicht hat. Zu diesem Zeitpunkt wird die Säurerezirkulation gestoppt, und die Säure wird bei gleichzeitiger Zuführ von kaltem Waschwasser in einem gesonderten Tank gesammelt. Das Waschwasser wird ca. 20 Minuten durch die Säule rezirkuliert. Dann wird die Rezirkulation des Wassers gestoppt. Das Wasser wird in einem Tank gesammelt, während gleichzeitig die Zufuhr von kaltem Regenerierungspuffer gestartet wird. Dieser Puffer wird 12 - 16 Stunden lang bei +5 °C durch die Säule rezirkuliert. Dann wird die Puffer-Rezirkulation gestoppt. Der Puffer wird in einem Tank gesammelt, während gleichzeitig die Zufuhr der Substratlösung gestartet wird, nachdem die Säule auf +25 °C temperiert wurde.

Das Ergebnis von vier konsekutiven Verwendurgen des Betreibens einer solchen Säule im Labormaßstab ist in dem Beispielen 7-10 gegeben. Aus den Ergebnissen erkennt man, daß ein FBR eine günstigere Steuerung der Reaktion in Richtung Synthese gestattet, als dies im Rührkessel möglich ist. Allerdings involviert ein FBR einen erheblich höheren technischen Aufwand, mit den hiermit stets verbundenen höheren Risiken von Fehlsteuerung.

### Fazit

Die Ergebnisse unserer Versuche im FBR und im Rührkessel zeigen die technische Anwendbarkeit des erfinderischen Prinzips der Produktisolierung in Anwesenheit des Biokatalysators bei pH 1,0 und die Regenerierung des Biokatalysators durch Inkubation in Neutralpuffer. Bei dem vorliegenden Anwendungsgebiet, nämlich der enzymatischen Synthese von Betalactam-Antibiotika, war es nicht sinnvoll, zu untersuchen, ob dieses Prinzip auch im alkalischen pH-Bereich funktioniert.

| **Im Text gebrauchte Abkürzungen:** | | | |
|---|---|---|---|
| I-E | Immobilisiertes Enzym | RLR | Rühr-Laborreaktor |
| O-ST | operationale Stabilität der Aktivität eines I-E | HPLC | High Performance Liquid Chromatography |
| DEI-Wasser | Deionisiertes Wasser | | |
| g | Gramm | NMR | Nuclear Magnetic Resonance (Kernresonanz) |
| ml | Milliliter | pH | negativer dekadischer Logarythmus der molaren Wasserstoffionen-Konzentration |
| 6-APA | 6-Aminopenicillansäure | | |
| 7-ADCA | 7-Amino-Desacetoxycephalosporansäure | PHE-GLY | D-Phenylglycin |
| | | PHE-GLY-ME | D-Phenylglycin-Methylester |
| PcA | Penicillinamidase | | |
| E.coli | Escherichia Coli | | |
| FBR | Festbett-Reaktor | | |

### Beispiele

### Beispiel 1

### Herstellung der Substratlösung für RLR-Versuche

Zu 20 ml deionisiertem Wasser wurden 4,3 g 6-Aminopenicillansäure (6-APA) und 10 g D-Phenylglycin-Methylester-Hydrochlorid gegeben. Unter Rühren und Kühlung auf 18 - 22 °C werden ca. 38 ml Ammoniak (1,25 molare wässerige Lösung) zugetropft, so daß der pH-Wert des Gemisches einen Wert von pH 8,0 zu Beginn und einem pH-Wert von 7,0 zu Ende des Auflösevorgangs nicht übersteigt. Auf diese Weise werden ca. 65 ml Substratlösung erhalten. Die Lösung wird gewogen und nach Entnahme einer kleinen Menge (ca. 1 g) für analytische Zwecke quantitativ für 1 enzymatische Synthese im Rührreaktor verwendet.

### Beispiel 2

### Enzymatische Synthese im Rühr-Laborreaktor (RLR) / Erste Umsetzung

Die Substratlösung von Beispiel 1 wird auf 20 Gramm (Feuchtgewicht) von an Eupergit C® immobilisierter Penicillinamidase unter Rühren gegeben. Hierfür wurde PharmaKAT-PcA, ein Verkaufsprodukt der Firma FZB Biotechnik GmbH, Berlin, eingesetzt.
Die Suspension wurde 180 Minuten lang bei 23 - 25 °C gerührt, wobei der pH-Wert durch Zugabe kleinerer Mengen an 1,25 molarem Ammoniak konstant auf pH 7,0 gehalten wurde.

Dann wurde das Gemisch auf 0 °C abgekühlt und anschließend unter Rühren und Kühlen solange tropfenweise 2,5 molare (ca. 25%ige ) Schwefelsäure zugegeben, bei ständiger Kühlung auf 0 °C und unter genauer pH-Kontrolle, bis ein konstanter pH-Wert von 1,0 erreicht war. Dann wurde diese Lösung noch weitere 10 - 20 Minuten lang unter Kühlung auf 0 °C gerührt und anschließend über das Bodensieb des Reaktors abgesaugt. Von dem Filtrat wurde 1,0 g abgezweigt für analytische Bestimmungen. Die Hauptmenge wurde zur Isolierung der Reaktionsprodukte eingesetzt. Hierzu wurde der pH-Wert durch Zugabe von konzentriertem Ammoniak auf pH 5,1 gebracht, wobei die Temperatur des Gemisches durch Kühlung stets auf 0 °C gehalten wurde. Die Mischung wurde 2 Stunden lang bei 0 °C und pH 5,1 gerührt. Hierbei fiel ein kristallines Produkt aus. Es wurde abfiltriert und mit kaltem Wasser gewaschen, scharf abgesaugt und über Nacht im Vakuum bei +30 °C getrocknet: 7,67 g. Dieses Produkt wurde mit HPLC auf seinen Gehalt an Ampicillin-Trihydrat untersucht; Ergebnis: 51% Ampicillin-Trihydrat, entsprechend 3,9 g; dies sind 9,67 mMol, was einer Ausbeute von 48% bezogen auf die Menge an eingesetzter 6-APA entspricht.

Das auf dem Filter im RLR verbliebene immobilisierte Enzym wurde in 100 ml kaltem DEI-Wasser suspendiert und unter Rühren mit kaltem 1,25 molaren Ammoniak unter Kühlung auf einen pH-Wert von 6,5 bis 7,0 gebracht, 10 Minuten weitergerührt und abgesaugt. Dann wurden 100 ml kalter K-Phosphatpuffer (0,1 molar, pH 7,5) zugegeben. Die Mischung wurde bei +5 °C 3 Stunden lang sanft gerührt und dann ohne weiteres Rühren 12 Stunden lang bei +5 °C im Reaktor gelassen.

### Beispiel 3

### Enzymatische Synthese im RLR / Zweite Umsetzung

Der Phosphatpuffer aus Beispiel 2 wurde vom I-E abgesaugt, wobei I-E auf dem Bodensieb im Reaktor verbleibt. Dort wurde es mit 50 ml Wasser von 23 °C gewaschen und abgesaugt.
Anschließend wurde Substrat nach Beispiel 1 auf das I-E gegeben.
Die Suspension von I-E in der Substratlösung wurde 180 Minuten lang bei 23 - 24 °C gerührt. Hierbei und bei der anschließenden weiteren Durchführung wurde exakt in gleicher Weise verfahren, wie für Beispiel 2 beschrieben.
Ergebnis: 7,46 g kristallines Produkt.
Gehalt an Ampicillin-Trihydrat: 3,4 g, entsprechend 42% Ausbeute bezogen auf 6-APA.

### Beispiel 4

### Enzymatische Synthese im RLR / Dritte Umsetzung

Der Phosphatpuffer aus Beispiel 3 wurde von I-E abgesaugt, und anschließend wurde exakt in der gleichen Weise verfahren, wie für Beispiel 2 beschrieben.
Ergebnis: 7,3 g kristallines Produkt;
Gehalt an Ampicillin-Trihydrat 4,0 g, entsprechend 50% Ausbeute bezogen auf 6-APA.

### Beispiel 5

### Enzymatische Synthese im RLR / Vierte Umsetzung

Der Phosphatpuffer aus Beispiel 4 wurde vom I-E abgesaugt, und anschließend wurde exakt in der gleichen Weise verfahren, wie für Beispiel 2 beschrieben.
Ergebnis: 7,32 g kristallines Produkt.
Gehalt an Ampicillin-Trihydrat 3,2 g, entsprechend 40% Ausbeute bezogen auf 6-APA.

Flächenverhältnis von Ampicillin-Trihydrat und D-Phenylglycin im HPLC-Diagramm als relatives Maß für das Verhältnis von Synthese zu Hydrolyse betrug 1,1.

### Beispiel 6

### Herstellung der Substratlösung für Versuche im Festbett-Reaktor (FBR)

16,1 g 6-APA und 15,0g D-Phenylglycin-Methylester-Hydrochlorid wurden in 190 ml DEI-Wasser unter Rühren bei 24 - 25 °C durch vorsichtige Zugabe von Ammoniak (2,4 molar) aufgelöst und auf einen pH-Wert von 7,0 gebracht. Hierfür wurden insgesamt ca. 45 ml Ammoniak zugegeben.

### Beispiel 7

### Enzymatische Synthese im Festbettreaktor (FBR) / Erste Verwendung

Penicillinamidase immobilisiert an Eupergit C® (PharmaKAT-PcA) wurde in DEI-Wasser suspendiert und in einen FBR zu einem Bettvolumen von 110 ml eingefüllt. Der Reaktor war aus rostfreiem Stahl hergestellt. Sein Durchmesser betrug 43 Millimeter (mm), seine Höhe 150 mm. Die Füllhöhe des Festbettes betrug 76 mm.

Auf dieses Festbett wurde Substratlösung (nach Beispiel 6) von oben gegeben und bei einer Flußrate von 2,5 ml pro Minute durch das Festbett geleitet und fraktionsweise gesammelt. In einzelnen Fraktionen wurde der Gehalt an Ampicillin, 6-APA, D-Phenylglycin-Methylester und D-Phenylglycin mit HPLC bestimmt. Einzelergebnis: siehe Tabelle. Flächenverhältnis im HPLC-Diagramm von Ampicillin zu D-Phenylglycin als relatives Maß von Synthese/Hydrolyse betrug 0,83.
Nach Sammeln von 150 ml umgesetzter Substratlösung wurde die Zufuhr von Substratlösung gestoppt und soviel 1,25 molare Schwefelsäure bei einer Temperatur von 0°C durch das Festbett geleitet, bis am Ausgang des Festbett-Reaktors ein pH-Wert von 1,0 erreicht war. Sobald die pH-Messung am Festbett-Ausgang einen pH-Wert von 1,0 für einen Zeitraum von 30 Minuten zeigte, wurde die Zufuhr an Schwefelsäure gestoppt. Nun wurde nach einem Waschschritt mit kaltem Wasser die Zufuhr von Kaliumphosphatpuffer (0,1 molar, pH 7,5) bei einer Temperatur von +5°C gestartet. Sobald am Ausgang des Reaktors ein pH-Wert von 7,5 erreicht war, wurde der Pufferstrom durch die Säule rezirkuliert, d. h. im Kreis geführt, und zwar über 12 Stunden bei einer Flußrate von 1 ml/min und einer Temperatur von +5 °C.
Auf diese Weise wurde der Biokatalysator für die nächstfolgende Verwendung zur Synthese von Ampicillin regeneriert.

### Beispiel 8

### Enzymatische Synthese im Festbett-Reaktor (FBR) / Zweite Verwendung

Das durch Inkubation mit Puffer regenerierte immobilisierte Enzym aus Beispiel 7 wurde mit DEI-Wasser im FBR gewaschen. Dann wurde auf dieses Festbett Substratlösung (nach Beispiel 6) von oben gegeben und bei einer Flußrate von 1,7 ml pro Minute durch das Festbett geleitet und am Reaktorausgang fraktionsweise gesammelt. In einzelnen Fraktionen wurde der Gehalt an Ampicillin, 6-APA, D-Phenylglycin-Methylester und D-Phenylglycin mit HPLC bestimmt.
Einzelergebnis: siehe Tabelle.
Relatives Maß von Synthese zu Hydrolyse (entsprechend Flächenverhältnis im HPLC-Diagramm) betrug 1,9.

Nach Sammeln von 150 ml umgesetzter Substratlösung wurde die Zufuhr an Substratlösung gestoppt. Die weitere Verfahrensweise war identisch mit der aus Beispiel 7.

### Beispiel 9

### Enzymatische Synthese im Festbett-Reaktor (FBR) / Dritte Verwendung

Es wurde das regenerierte immobilisierte Enzym aus Beispiel 8 eingesetzt und sonst in der gleichen Weise verfahren, wie für Beispiel 8 beschrieben, mit dem Unterschied, daß die Flußrate 1 ml pro Minute betrug und die Substratzufuhr nach Sammeln von 170 ml gestoppt wurde.
Einzelergebnis: siehe Tabelle.
Relatives Maß von Synthese zu Hydrolyse (entsprechend Flächenverhältnis im HPLC-Diagramm) betrug 2,2.

### Beispiel 10

### Enzymatische Synthese im Festbett-Reaktor (FBR) / Vierte Verwendung

Es wurde das regenerierte immobilisierte Enzym aus Beispiel 9 eingesetzt und sonst in der gleichen Weise verfahren, wie für Beispiel 9 beschrieben, mit dem Unterschied, daß die Substratzufuhr nach Sammeln von 304 ml gestoppt wurde.
Einzelergebnis: siehe Tabelle.
Relatives Maß von Synthese zu Hydrolyse (entsprechend Flächenverhältnis im HPLC-Diagramm): 2,1.

### Beispiel 11

### Durchführung der HPLC-Untersuchungen: Methode und Material

Für die HPLC-Untersuchungen wurde das Gerät HP 1100 der Firma Hewlett-Packard eingesetzt.

### Probenvorbereitung

1g Lösung (bei den Rührreaktor-Versuchen nach Solubilisierung durch H₂SO₄; bei den FBR-Versuchen direkt aus dem Säuleneluat) wurde mit Phosphatpuffer (25 millimolar; pH 6,5) auf ein Endvolumen von 25 ml verdünnt.

Bei Untersuchung fester Proben wurden 50 mg Produkt in 50 ml Phosphatpuffer (25 millimolar; pH 6,5) gelöst.

### Zubereitung der Standard-Lösungen

Jeweils 50 mg Ampicillin-Trihydrat oder 50 mg D-Phenylglycin oder 50 mg 6-APA oder 50 mg D-Phenylglycin-Methylester-Hydrochlorid wurden in jeweils 50 ml Phosphatpuffer (25 millimolar, pH 6,5) gelöst.

### Methode: Gradientenmethode

Eluent A: 25 millimolarer Phosphatpuffer (pH 6,5)
Eluent B: Acetonitril
Flußrate: 1 ml/min
Wellenlänge: 215 nm
Injektionsvolumen: 20 Mikroliter
Analysendauer: 20 Minuten
HPLC-Säule: MAXIL 5C18 (250 x 4,60 mm), 5 Micron

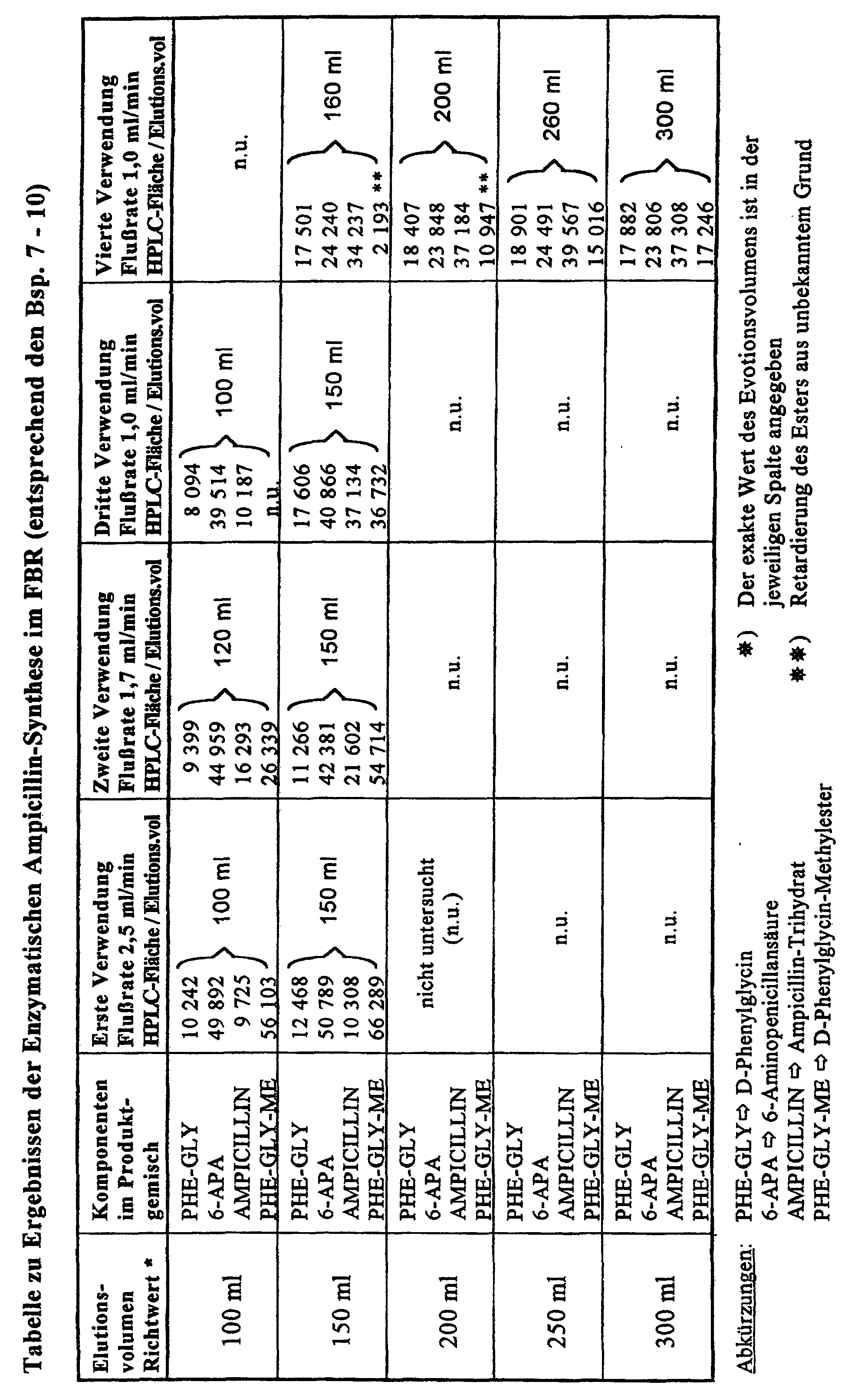

## Patentansprüche

1. Verfahren zur Herstellung von aminogruppenhaltigen Betalactam-Antibiotika aus ihren Aminobetalactam-Komponenten und den entsprechenden aminogruppenhaltigen acylierenden Seitenkettenkomponenten über die biokatalytische Wirkung eines an Trägerpartikel kovalent immobilisierten Enzyms,
**dadurch gekennzeichnet, daß**
- die Reaktionsprodukte bei pH 1,0 und in einem Temperaturbereich von 0°C bis +5°C in Gegenwart des immobilisierten Enzyms Penicillinamidase aus E. coli solubilisiert werden
und
- dann von dem immobilisierten Enzym abgetrennt werden,
- welches anschließend durch Behandlung mit einem Puffer neutralen pH-Bereichs bei einer Temperatur von ca. +5°C in Bezug auf seine Aktivität regeneriert wird.

2. Verfahren nach Anspruch 1,
**dadurch gekennzeichnet, daß**
- das herzustellende Antibiotikum Ampicillin,
- die Aminobetalactam-Komponente 6-Aminopenicillansäure
und
- die acylierende Seitenkettenkomponente D-Phenylglycin-Methylester oder D-Phenylglycinamid ist.

3. Verfahren nach Anspruch 1,
**dadurch gekennzeichnet, daß**
- das herzustellende Betalactam-Antibiotikum Cephalexin,
- die Aminobetalactam-Komponente 7-Amino-Desacetoxycephalosporansäure (7-ADCA)
und
- die acylierende Seitenkettenkomponente D-Phenylglycin-Methylester oder D-Phenylglycinamid ist.

4. Verfahren nach Anspruch 1,
**dadurch gekennzeichnet, daß**
- das herzustellende Betalactam-Antibiotikum Cefaclor,
- die Aminobetalactam-Komponente 3-Chloro-7-Amino-Desacetoxycephalosporansäure
und
- die acylierende Seitenkettenkomponente D-Phenylglycin-Methylester oder D-Phenylglycinamid ist.

5. Verfahren nach Anspruch 1,
**dadurch gekennzeichnet, daß**
- das herzustellende Betalactam-Antibiotikum Amoxicillin,
- die Aminobetalactam-Komponente 6-Aminopenicillansäure
und
- die acylierende Seitenkettenkomponente D-p-Hydroxyphenylglycin-Methylester oder D-p-Hydroxyphenylglycinamid ist.

6. Verfahren nach Anspruch 1,
**dadurch gekennzeichnet, daß**
- das herzustellende Betalactam-Antibiotikum Cefadroxil,
- die Aminobetalactam-Komponente 7-Amino-Desacetoxycephalosporansäure (7-ADCA)
und
- die acylierende Seitenkettenkomponente D-p-Hydroxyphenylglycin-Methylester oder D-p-Hydroxyphenylglycinamid ist.

7. Verfahren nach Anspruch 1,
**dadurch gekennzeichnet, daß**
die Trägerpartikel eine makroporöse Struktur bei einem Teilchendurchmesser von 10 - 1000 Micrometer haben.

8. Verfahren nach Anspruch 7,
**dadurch gekennzeichnet, daß**
die Trägerpartikel Oxirangruppen zur kovalenten Immobilisierung des Enzyms haben.

9. Verfahren nach Anspruch 8,
**dadurch gekennzeichnet, daß**
die oxirangruppenhaltigen makroporösen Trägerpartikel aus synthetischen Polymeren bestehen.

10. Verfahren nach Anspruch 8,
**dadurch gekennzeichnet, daß**
die oxirangruppenhaltigen makroporösen Trägerpartikel aus anorganischem Material bestehen.

11. Verfahren nach Anspruch 10,
**dadurch gekennzeichnet, daß**
die anorganischen Trägerpartikel aus porösem Glas bestehen.

12. Verfahren nach Anspruch 1,
**dadurch gekennzeichnet, daß**
- alle verfahrensrelevanten Schritte in einem Rührreaktor ausgeführt werden,
- der mit einem Bodensieb versehen ist.

13. Verfahren nach Anspruch 1,
**dadurch gekennzeichnet, daß**
alle verfahrensrelevanten Schritte in einem Festbettreaktor durchgeführt werden.

14. Verfahren nach Anspruch 1,
**dadurch gekennzeichnet, daß**
zur Solubilisierung der Reaktionsprodukte fünfprozentige bis fünfzigprozentige Schwefelsäure in einer Menge zum dem gekühlten Reaktionsproduktgemisch zugegeben wird, bis in diesem ein pH-Wert von 1,0 erreicht ist.

15. Verfahren nach Anspruch 1,
**dadurch gekennzeichnet, daß**
der zur Regenerierung des immobilisierten Enzyms verwendete Puffer Phosphatpuffer von einer Molarität von 0,1 bis 1,0 und einem pH-Wert von 6,5 bis 8,0 ist.

## Claims

1. Process for preparing amino group-containing beta-lactam antibiotics from their amino-beta-lactam components and the appropriate amino group-containing acylating side-chain components via the biocatalytic effect of an enzyme which is covalently immobilized on carrier particles, **characterized in that**
- the reaction products are solubilized at pH 1.0 and in a temperature range from 0°C to +5°C in the presence of the immobilized enzyme penicillin amidase from E. coli
and
- are then separated from the immobilized enzyme,
- which is subsequently regenerated in relation to its activity by treatment with a buffer of neutral pH range at a temperature of about +5°C.

2. Process according to Claim 1, **characterized in that**
- the antibiotic to be prepared is ampicillin,
- the amino-beta-lactam component is 6-aminopenicillanic acid
and
- the acylating side-chain component is D-phenyl-glycine methyl ester or D-phenylglycinamide.

3. Process according to Claim 1, **characterized in that**
- the beta-lactam antibiotic to be prepared is cephalexin,
- the amino-beta-lactam component is 7-amino-deacetoxycephalosporanic acid (7-ADCA)
and
- the acylating side-chain component is D-phenyl-glycine methyl ester or D-phenylglycinamide.

4. Process according to Claim 1, **characterized in that**
- the beta-lactam antibiotic to be prepared is cefaclor,
- the amino-beta-lactam component is 3-chloro-7-aminodeacetoxycephalosporanic acid
and
- the acylating side-chain component is D-phenyl-glycine methyl ester or D-phenylglycinamide.

5. Process according to Claim 1, **characterized in that**
- the beta-lactam antibiotic to be prepared is amoxicillin,
- the amino-beta-lactam component is 6-aminopenicillanic acid
and
- the acylating side-chain component is D-p-hydroxyphenylglycine methyl ester or D-p-hydroxyphenylglycinamide.

6. Process according to Claim 1, **characterized in that**
- the beta-lactam antibiotic to be prepared is cefadroxil,
- the amino-beta-lactam component is 7-amino-deacetoxycephalosporanic acid (7-ADCA)
and
- the acylating side-chain component is D-p-hydroxyphenylglycine methyl ester or D-p-hydroxyphenylglycinamide.

7. Process according to Claim 1, **characterized in that** the carrier particles have a macroporous structure with a particle diameter of 10-1 000 micrometres.

8. Process according to Claim 7, **characterized in that** the carrier particles have oxirane groups for covalent immobilization of the enzyme.

9. Process according to Claim 8, **characterized in that** the oxirane group-containing macroporous carrier particles consist of synthetic polymers.

10. Process according to Claim 8, **characterized in that** the oxirane group-containing macroporous carrier particles consist of inorganic material.

11. Process according to Claim 10, **characterized in that** the inorganic carrier particles consist of porous glass.

12. Process according to Claim 1, **characterized in that**
- all process-relevant steps are carried out in a stirred reactor,
- which is provided with a bottom screen.

13. Process according to Claim 1, **characterized in that** all process-relevant steps are carried out in a fixed bed reactor.

14. Process according to Claim 1, **characterized in that** for solubilization of the reaction products five per cent strength to fifty per cent strength sulphuric acid is added in an amount to the cooled reaction product mixture until a pH of 1.0 is reached in the latter.

15. Process according to Claim 1, **characterized in that** the buffer used for regenerating the immobilized enzyme is phosphate buffer with a molarity of from 0.1 to 1.0 and a pH of from 6.5 to 8.0.

## Revendications

1. Procédé en vue de la fabrication d'antibiotiques de bêta-lactame contenant des groupements amino à partir de leurs composants d'amino-bêta-lactames et des composés à chaînes latérales acylants contenant des groupements amino par l'intermédiaire de l'action bio-catalytique d'une enzyme immobilisée de manière covalente sur une particule vecteur, **caractérisé en ce que**
- les produits de réaction sont solubilisés à un pH de 1,0 et dans un domaine de température de 0°C à +5°C en présence de l'enzyme immobilisée pénicillinamidase provenant de E. Coli,
et
- sont alors séparés de l'enzyme immobilisée,
- qui est régénérée ensuite, pour ce qui est de son activité, par traitement à l'aide d'un tampon dans le domaine de pH neutre à une température d'environ +5°C.

2. Procédé selon la revendication 1, **caractérisé en ce que**
- l'antibiotique à fabriquer est l'ampicilline,
- le composant d'amino-bêta-lactame est l'acide 6-aminopénicillanique
et
- le composant à chaînes latérales acylant est l'ester méthylique de la D-phénylglycine ou l'amide de la D-phénylglycine.

3. Procédé selon la revendication 1, **caractérisé en ce que**
- l'antibiotique de bêta-lactame à fabriquer est la Céphalexine,
- le composant d'amino-bêta-lactame est l'acide 7-amino-désacétoxycéphalosporinique (7-ADCA)
et
- le composant à chaînes latérales acylant est l'ester méthylique de la D-phénylglycine ou l'amide de la D-phényiglycine.

4. Procédé selon la revendication 1, **caractérisé en ce que**
- l'antibiotique de bêta-lactame à fabriquer est le Cefaclor,
- le composant d'amino-bêta-lactame est l'acide 3-chloro-7-aminodésacétoxycéphalosporinique
et
- le composant à chaînes latérales acylant est l'ester méthylique de la D-phénylglycine ou l'amide de la D-phénylglycine.

5. Procédé selon la revendication 1, **caractérisé en ce que**
- l'antibiotique de bêta-lactame à fabriquer est l'amoxycilline,
- le composant d'amino-bêta-lactame est l'acide 6-aminopénicillanique
et
- le composant à chaînes latérales acylant est l'ester méthylique de la D-p-hydroxyphénylglycine ou l'amide de la D-p-hydroxyphénylglycine.

6. Procédé selon la revendication 1, **caractérisé en ce que**
- l'antibiotique de bêta-lactame à fabriquer est le Cefadroxil,
- le composant d'amino-bêta-lactame est l'acide 7-amino-désacétoxycéphalosporinique (7-ADCA)
et
- le composant à chaînes latérales acylant est l'ester méthylique de la D-p-hydroxyphénylglycine ou l'amide de la D-p-hydroxyphénylglycine.

7. Procédé selon la revendication 1, **caractérisé en ce que** les particules vecteurs ont une structure macro-poreuse pour un diamètre de particules de 10-1000 micromètres.

8. Procédé selon la revendication 7, **caractérisé en ce que** les particules vecteurs ont des groupements oxiranne en vue de l'immobilisation covalente de l'enzyme.

9. Procédé selon la revendication 8, **caractérisé en ce que** les particules vecteurs macroporeuses contenant des groupements oxiranne se composent de polymères synthétiques.

10. Procédé selon la revendication 8, **caractérisé en ce que** les particules vecteurs macroporeuses contenant des groupements oxiranne se composent d'un matériau inorganique.

11. Procédé selon la revendication 10, **caractérisé en ce que** les particules vecteurs inorganiques se composent de verre poreux.

12. Procédé selon la revendication 1, **caractérisé en ce que** toutes les étapes pertinentes au procédé sont effectuées dans un réacteur à agitateur, qui est pourvu d'un tamis de fond.

13. Procédé selon la revendication 1, **caractérisé en ce que** toutes les étapes pertinentes au procédé sont effectuées dans un réacteur à lit fixe.

14. Procédé selon la revendication 1, **caractérisé en ce que** l'on ajoute au mélange de produits de réaction refroidi, en vue de la solubilisation des produits de réaction, de l'acide sulfurique de cinq à cinquante pour cent dans une quantité telle que l'on parvienne à atteindre dans ce mélange une valeur de pH de 1,0.

15. Procédé selon la revendication 1, **caractérisé en ce que** le tampon utilisé en vue de la régénération de l'enzyme immobilisée est un tampon aux phosphates d'une molarité de 0,1 à 1,0 et d'une valeur de pH de 6,5 à 8,0.
